# EUROPEAN PATENT APPLICATION

(11) **EP 2 609 909 A1**
(43) Date of publication of application: **03.07.2013**
(21) Application number: 11196236.1
(22) Date of filing: 30.12.2011
(51) Int. Cl.: A61K 9/00

(54) **Mozavaptan formulations**

(71) Applicant: Deva Holding Anonim Sirketi, 34303 Istanbul (TR)
(72) Inventor: Haas, Philipp Daniel, 34303 Istanbul (TR); Koc, Fikret, 34303 Istanbul (TR); Sivasligil, Ramazan, 34303 Istanbul (TR); Kandemir, Levent, 34303 Istanbul (TR); Ilhan, Suna Ayse, 34303 Istanbul (TR)

(57) **Abstract**

The present invention is related to easy to use dosage forms of mozavaptan or a pharmaceutically acceptable salt thereof comprising orally disintegrating tablets, effervescent tablets, granules in sachets, dry syrup and manufacturing methods thereof.

## Description

### Technical Field

The present invention is related to easy to use dosage forms of mozavaptan or a pharmaceutically acceptable salt thereof comprising orally disintegrating tablets, effervescent tablets, granules in sachets, dry syrup and manufacturing methods thereof.

### Background Art

Sodium is the major extracellular electrolyte and is responsible for maintaining extracellular osmolality and trans-membrane electrical and chemical potentials.

Hyponatremia is a serious electrolyte disorder characterized by a subnormal concentration of sodium in the blood and it is manifested by a range of neurological symptoms which can lead to seizure, obtundation, hypoxia and death if it is left untreated.

Mozavaptan is vasopressin receptor antagonist used for treatment caused by syndrome of inappropriate anti-diuretic hormone (SIADH) due to anti-diuretic hormone producing tumors. Mozavaptan is freely soluble in methanol.

Mozavaptan is chemically described as 5-(Dimethylamino)-1-[4-(2-methylbenzamido)benzoyl]-2,3,4,5-tetrahydro-1 H-benzazepine, and it has the following structural formula:

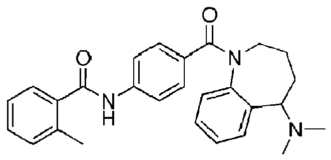

WO 91/05549 A (OTSUKA PHARMACEUTICAL COMPANY LIMITED) 02.05.1991 firstly discloses mozavaptan.

Generally, pediatric, elderly, geriatric, bedridden, or developmentally disabled persons face difficulty in swallowing conventional dosage forms because of dysphasia. Moreover in some disease conditions such as motion sickness or persistent nausea, repeated emesis or sudden episodes of allergic attack, or coughing it becomes difficult to swallow conventional tablets.

Recently, a vast variety of pharmaceutical research is focused on increasing patient compliance and is directed at developing new dosage forms. Among dosage forms for facilitating the ease of medication, the concept of orally disintegrating dosage form has gained considerable attention to provide patient adherence in treatment.

Recent market studies show that orally disintegrating tablet is designated as a preferred alternative dosage form to conventional ones due to better patient compliance in patient with swallowing difficulties.

Travelling patients who do not have access to water also prefer orally disintegrating tablets due to the convenience to carry and ease to take.

In the present invention, all required tests and analyses are carried out and complies with the specific requirements of following pharmacopeias; USP (United States Pharmacopoeia), EP (European Pharmacopoeia), BP (British Pharmacopoeia).

European Pharmacopeia adopted the term orally disintegrating tablet as a tablet to be placed in the mouth where it disperses rapidly before swallowing and which disintegrates in less than 3 min.

More preferably, the pharmaceutical formulations are disintegrated in an oral cavity in less than two minutes and most preferably the pharmaceutical formulations are disintegrated in oral cavity in less than one minute.

Orally disintegrating tablets are also known as orodispersible, rapimelts, quick dissolving, rapid disintegrating, rapid dissolving, mouth dissolving, melt-in-mouth, mouth dispersing, fast mouth dissolving, fast melting, porous tablet, orodispersing tablets.

The performance of orally disintegrating tablet s depends on the technology. Conventional techniques including direct compression, freeze drying, spray drying, moulding, phase transition process, melt granulation, sublimation, mass extrusion, cotton candy processes are the most populars. Additionally, some other different patented techniques are developed for preparation of ODT's such as Zydis, Orasolv, Durasolv, Flashtab, Wow tab, Ora quick, Ziplet, Flashdose.

Other friendly dosage forms can be conveniently represented in the suitable form of orally disintegrating tablet, sachet, effervescent tablet and dry syrup to enhance the patient compliance and administration convenience in the treatment. Such forms of mozavaptan need to be reconstituted with water or other suitable liquid before use and may be administered as fully dissolved, dispersed or suspended in a solution.

Mozavaptan is known to have unpleasant taste which is aimed to be masked in the formulation.

However, few formulations of mozavaptan or a pharmaceutically acceptable salt thereof have been described in the past and none of these formulations discloses easy-to-use dosage forms and formulations of mozavaptan.

### Summary of invention

The present invention is related to easy to use dosage forms for mozavaptan or a pharmaceutically acceptable salt thereof comprising orally disintegrating tablets, effervescent tablets, granules in sachets, dry syrup or drug in solution and manufacturing methods thereof.

### Technical Problem

Difficulty in swallowing which is known as dysphagia is common among all age groups. Person with dysphagia or elderly people or paediatrics experience pain when trying to swallow traditional solid oral dosage forms and they frequently refuse taking solid medication.

Non compliance to prescription of hyponatremic patient may result in inefficient treatment or total failure of the treatment.

Orally disintegrating tablets are required to disintegrate and dissolve in the oral cavity to release the medication which comes into contact with taste buds. Hence pharmaceutically active ingredients that leave an unpleasant or bitter taste are needed to be masked in the oral cavity after administration.

A major element in orally disintegrating tablet formulation is good taste. Bitterness of the preparation leads to lack of patient compliance. Succesful taste masking is needed to hide bitterness and combining this with right production method results in a superior product.

On the other hand, building up appropriate pharmaceutical formulation and a related production method is a challenge to formulation scientist during product development phase. Inappropriate manufacturing methods cause problems, hinder the processibility, thereby bringing about unnecessary delays in production.

Although various conventional or patented manufacturing techniques have been developed lately by many companies for orally disintegrating tablet, development of oral disintegrating tablet of mozavaptan remain a changeling area.

Therefore, the development of a new manufacturing method which does not require any special investment is always needed.

The purpose of the present invention is to develop easy-to-use dosage forms for mozavaptan or a pharmaceutically acceptable salt thereof comprising orally disintegrating tablets, effervescent granules, granules in sachets, dry syrup and manufacturing methods thereof by improving content uniformity across presented formulations.

However, few formulations of mozavaptan have been described in the past and none of these formulations discloses easy-to-use dosage forms and formulations of mozavaptan.

### Solution to Problem

In order to enhance the patient compliance and convenience of administration in the treatment, patient friendly dosage forms are conveniently presented in the suitable form of oral disintegrating tablet, effervescent tablet, granules in sachet, and dry syrup. They are particularly designed to be administered to patients who have difficulties to swallowing capsules or tablets.

Presented dosage forms and formulations surpass previously presented problems arising in product formulation and administration of mozavaptan.

The present invention proposes oral disintegrating tablet and liquid oral pharmaceutical compositions which are desirable due to their ease in administration. Relating controllable manufacturing methods are also presented for each oral solid dosage forms.

### Description of embodiments

In the present invention, more accessible, patient compliant, easy to use dosage forms of mozavaptan or a pharmaceutically acceptable salt thereof including orally disintegrating tablets, granules in sachets, dry syrup are firstly developed by inventors. Presented easy-to-use dosage forms include an effective amount of mozavaptan or a pharmaceutically acceptable salt thereof and at least one pharmaceutically acceptable excipient.

The term "drug", "active" or "active pharmaceutical ingredient" as used herein includes a pharmaceutically acceptable and therapeutically effective amount of mozavaptan or pharmaceutically acceptable salts, stereoisomers and mixtures of stereoisomers, solvates (including hydrates), and/or esters thereof.

Mozavaptan hydrochloride is preferred.

In the first aspect of the present invention, orally disintegrating tablet dosage form of mozavaptan or a pharmaceutically acceptable salt thereof that dissolves or disperses rapidly when in contact with saliva is provided.

Oral disintegrating dosage forms include an effective amount of mozavaptan or a pharmaceutically acceptable salt thereof and at least one pharmaceutically acceptable excipient or mixture of excipients.

Orally disintegrating tablets are useful for elderly people, children and patients who have difficulties in swallowing tablets. Such peoples are unwilling and/or unable to swallow traditional solid dosage forms. This is especially the case of pharmaceuticals for paediatric or geriatric use. To solve this problem, tablets that disintegrate rapidly in the mouth were developed.

These oral disintegrating tablets or orally dissolving dosage forms are typically tablets and thin films that do not require water for swallowing.

According to this invention, oral disintegrating tablets dosage forms may be, but not limited to, rapidly disintegrating tablets, lingual strips, sublingual tablets, sublingual strips, oral mists, lyophilized wafers, granulated particles, spray, gums whatsoever.

An orally disintegrating tablet of mozavaptan or a pharmaceutically acceptable salt thereof may have many numerous shapes, such as dish-like, ellipsoid, rods, granules, blocks, cubes with rounded edges, or any other shape suitable for pharmaceutical administration.

Orally disintegrating tablets are evaluated to according to following features; wetting time, hardness, friability test, mechanical strength, uniformity of dispersion, water absorption ratio, taste/mouth sensation, in-vitro and in-vivo disintegration test, in-vitro dissolution test and stability studies and moisture uptake studies.

The time of disintegration of ODTs is generally less than one minute and it is desired to provide minimum disintegrating time in oral cavity. In this point, tablet hardness is very important.

The rate of disintegration of the ODT compositions of the present invention can be measured using various in vitro test methods, for example the USP <701> Disintegration Test. When using the USP <701> Disintegration Test, the rate of disintegration of ODT compositions is faster than that of conventional oral, for example about 60 seconds or less, about 30 seconds or less, about 20 seconds or less.

According to this invention, orally disintegrating tablet of mozavaptan hardness is in the range of 20 to 75 N. Quick disintegration is provided in 30 seconds if tablet hardness is in the range of 20 to 75N.

Mozavaptan orally disintegrating tablet comprises; mozavaptan or pharmaceutically acceptable salt thereof is in the range of from 1 % to 60, binder is in the range of from 0.1 % to 15 %, coating agent is in the range of from 0.1 % to 50 %, pore former is in the range of from 0.01 % to 5 %, disintegrant is in the range of from 10 % to 90 %, sweetener is in the range of from 0.1 % to 40 %, stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, by weight .In this formulation, it is possible that one or more elements, such as stabilizing agent, may not be used. In such a case, without some elements, inventors do not aim that such formulation is excluded the invention. Fewer multifunctional excipients can be used instead of more excipients.

The compressed tablets of mozavaptan have sufficient mechanical strength which allows them to be packed in normal push-through blister packages and does not require special packaging like peel-off blisters.

In the development of orally disintegrating tablet of mozavaptan, selection of proper packaging material and system is important for enhanced stability of the finished product.

In the present invention, the compositions of orally disintegrating tablet of mozavaptan can be prepared through using pharmaceutical technologies including, but not limited to, direct compression, wet granulation, spray drying, freeze-drying, heat molding ,tablet molding, sublimation, mass extrusion, cotton candy consumable thin film manufacture, tablet and the like.

The present invention disclose a method of preparing mozavaptan orally disintegrating tablet's compositions which comprise mozavaptan, a disintegrant, a sugar alcohol and/or a saccharide and a flavour wherein the method comprises (a)coating particles comprising mozavaptan with a taste masking layer (b) granule preparation with disintegrant, a portion of diluent and/or a saccharide (c)mixing the coated particles.

In the present invention, other easy-to-use dosage forms such as effervescent tablet, granules in sachet and dry syrup and related formulations of mozavaptan are developed by inventors. These dosage forms need to be reconstituted with water or other suitable liquid before use and may be administered as fully dissolved, dispersed or suspended in a solution. Hence they are particularly designed to be administered to patients who have difficulties to swallowing capsules or tablets.

Oral administration of the powder or tablet in solution or suspension or dispersion provides particularly several advantages over conventional oral solid dosage forms. As the drug product is already in solution at the time it is consumed, reduced localized contact of the drug in the upper gastrointestinal tract leads to less irritation and greater tolerability to patients.

In the second aspect of the present invention, effervescent tablet of mozavaptan or a pharmaceutically acceptable salt thereof is provided. Effervescent tablet dosage form includes an effective amount of mozavaptan or a pharmaceutically acceptable salt thereof and at least one pharmaceutically acceptable excipient or mixture of excipients.

Although effervescent tablets are generally added to an aqueous medium such as water prior to oral administration, they may be designed to disintegrate in the mouth.

Effervescent tablet are making use of a chemical reaction between a soluble acid and an alkali metal carbonate with the help of water to make CO₂ gas, which can give a fizzy taste in the mouth.

Typically, the acid is organic or mineral acid that is safe and approved for consumption and pharmaceutical and/or nutritional purposes which provides effective and rapid effervescent disintegration upon contact with water and the alkaline effervescent compound.

The acidity for the effervescent reaction can be obtained from three main sources: acids, acid anhydrides, and acid salts.

The food acids are most commonly used since they are generally regarded as safe (GRAS), they occur in nature and are ingestible. Example of food acid includes are, but not limited to, citric acid, tartaric acid, ascorbic acid, fumaric acid, malic acid, adipic and succinic acid, acetyl salicylic acid, nicotinic acid, mixtures thereof and the like. Citric anhydride, succinic anhyride, glutamic anhydride can be used as well.

The acid salt of the composition can be any suitable acid salt or any mixture of suitable salts. Examples of such a suitable acid salts or mixture of suitable salts includes sodium hydrogen phosphate, disodium dihydrogen pyrophospate, acid citrate salts, aminoacid hydrochlorides. Combinations thereof are possible.

In effervescent formulations, alkaline excipients can be used. The alkaline component can be any suitable alkaline effervescent compound those are safe and approved for pharmaceutical and/or nutritional purposes, and typically it is an organic base (e.g., an alkali metal carbonate). It provides an effective and rapid effervescent disintegration upon contact with water and the acid compound. The alkaline effervescing compound may be selected from the group consisting of carbonate salts, bicarbonate salts, and mixtures thereof.

Example of carbonate sources, but not limited to, sodium bicarbonate, sodium carbonate, potassium bicarbonate and potassium carbonate, sodium sesquicarbonate, sodium glycine carbonate, I-lysine carbonate, arginine carbonate and mixtures thereof. Alkaline earth metal carbonates and bicarbonates can be used but alkali metal carbonates and bicarbonates are preferred.

Any conventional effervescent agent for example a mixture of a substance which on dissolution in water forms an acid reacting solution, and a pharmaceutically acceptable carbonate can be used as effervescent agent.

The effervescent tablet formulation of mozavaptan comprises: mozavaptan or pharmaceutically acceptable salt thereof is in the range of from 1 % to 60 %, binder is in the range of from 0.1 % to 15 %, diluent is in the range of from 10 % to 90 sweetener is in the range of from 0.1 % to 40 %, stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, acidic and basic effervescent agents are in the range of from 0.1 % to 80%, disintegrant is in the range of from 0.5 % to 30 % by weight of pharmaceutical dosage form. Fewer multifunctional excipients can be used instead of more excipients.

In the third aspect of the present invention, granules in sachet dosage form of mozavaptan or a pharmaceutically acceptable salt thereof are provided.

Granules in sachet dosage form include an effective amount of mozavaptan or a pharmaceutically acceptable salt thereof and at least one pharmaceutically acceptable excipient or mixture of excipients.

As used, a sachet is a single dose of a pharmaceutical formulation. The formulation is preferably in the form of a particulate material, sphere, pellets, particle or granulate.

Alternatively, powder, granulate, pellets, spheres, particles of sachet may be in effervescent form or mixtures thereof. The sachet can be formulated as a powder, granulate, pellets, spheres, particles or effervescent powder, effervescent granulate, effervescent pellets, effervescent spheres, effervescent particles or mixtures.

Presented powder, granulate, pellets, spheres, particles or their mixtures of sachet may be formulated for reconstitution with water or another suitable liquid. Said powder, granulate, pellets, spheres, particles or their mixtures may be ready to use sachet's formulation which can be consumed directly from the packet.

For the purposes of the present invention a "sachet" is referred herein to any suitable single serving pack, a container, a package, an envelope or a bag and the like. Preferably the sachet is sealed and disposable.

The sachet may be made up from any suitable material, such as plastic, metal foil, paper, and combination thereof. The sachet includes a perforated region or a nick in the edge of the sachet for ease of tearing.

The sachet may be launched and sold individually or may be launched or sold in pack of two or more sachets.

The sachet formulation comprises: mozavaptan or pharmaceutically acceptable salt thereof is in the range of from 1 % to 60 %, binder is in the range of from 0.1 % to 15 %, diluents is in the range of from 10 % to 90 %, sweetener is in the range of from 0.1 % to 40 %, stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, disintegrant is in the range of from 0.5 % to 30 % by weight of pharmaceutical dosage form. Fewer multifunctional excipients can be used instead of more excipients.

In the fourth aspect of the present invention, dry syrup dosage form of mozavaptan or a pharmaceutically acceptable salt thereof is provided.

A dry syrup preparation means "preparation with water which is dissolved or suspended before use". The powder is provided in a suitable container such as glass or polyethylene bottle to which prior to use prescribed amount of water is added to give dispersion. The desired amount of the dispersion is taken orally by the patient according to his or her need.

Dry syrup formulation comprises dry powder or granules or pellets or mixtures thereof.

In presented easy-to-use dosage forms of mozavaptan or a pharmaceutically acceptable salt thereof including orally disintegrating tablet, effervescent tablet, granules in sachet, dry syrup, taste is need to be masked in the oral cavity. A patient friendly, pleasant taste a mouth feel is achieved by inventors in the presented formulations.

Taste masking aims to prevent the drug substance from interaction with taste buds.

This invention provides taste masked easy-to-use dosage forms.

Current taste-masking techniques employed for taste masking includes applying polymer coating, use of flavour enhancer, complexation with ion exchange resin, inclusion complex formation with cyclodextrin.

In the present invention, a taste masking layer may be created to mask bitter taste of mozavaptan.

The taste masking layer may comprise one or more coating agents. The taste masking layer can further include additional excipient or excipients such as, but not limited to, pore former.

In easy to use dosage forms, one or more taste masking excipients such as flavors, sweeteners, acidic amino acids, lipids, and surfactants can further be used.

The taste-masking layer (as described herein) can be applied to the mozavaptan or a pharmaceutically acceptable salt thereof particles by any suitable method, for example fluidized bed coating methods.

Taste masking coating solution may comprise; (a)an inorganic acid or mixtures of inorganic acids or;(b) an organic acid or mixtures of organic acids or;(c) mixture of organic acid(s) and inorganic acid(s) or; (d) an organic solvent or mixtures of organic solvents or; (e) mixtures of one or more organic solvents and any alternative (a) to (e) or (f) de-ionized water and any alternative (a) to (e) or (g) de-ionized water and a coating agent or mixtures of coating agents. (h) Optionally and additionally an excipient or mixtures of excipients.

There are number of technologies available which evaluate objectively taste. Taste sensor, taste chip, taste sensing system, electronic sensor, and electronic tongue rank as the most popular applications. Taste of mozavaptan or a pharmaceutically acceptable salt thereof may be masked and assessed by mentioned taste sensors.

According to this invention, easy to use dosage forms comprising orally disintegrating tablet, granules in sachet, dry syrup, drug in solution may include one or more pharmaceutically acceptable excipients, carriers, or diluents. In fast disintegrating or fast dissolving formulations, surfactants, diluents, sweeteners, disintegrants, binders, lubricants, glidants, colorants, flavors ,mixtures thereof and the like can be used.

In case of easy to use dosage forms for mozavaptan or a pharmaceutically acceptable salt thereof formulations, sugar-based excipients, super-disintegrating agents, effervescent agents and further suitable excipients can be used.

Patient compliance improvizing agents can be from artificial or natural origin or mixtures thereof. Their function may vary such as sweeteners, flavour enhancers, taste masking agents etc.

Acceptable excipients are, but not limited to, calcium sulfate, starch, mannitol, kaolin, sorbitol, xylitol, sodium chloride, sodium bicarbonate, citric acid, powdered cellulose derivatives, microcrystalline cellulose, pullulan, silicified microcrystalline cellulose, ammonium bicarbonate, carrageenan, carbohydrates such as Pharmaburst™, magnesium carbonate, tribasic calcium phosphate, calcium sulfate, magnesium oxide, poloxamer,gums, hydroxypropyl methylcellulose, gelatin, mixtures thereof, and the like .

Diluents may be, but not limited to, mannitol, sorbitol, xylitol, microcrystalline cellulose, silicified microcrystalline cellulose, hydroxypropyl methylcellulose, hydroxypropyl cellulose , pullulan and fast dissolving carbohydrates such as Pharmaburst™, mixtures thereof and the like.

Glidants may be, but not limited to, silicon dioxide, colloidal silicon dioxide, calcium silicate, magnesium silicate, magnesium trisilicate, talc, starch, mixtures thereof or whatsoever.

Binders are, but not limited to, sodium alginate,cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth,sorbitol, acacia, alginates, carrageenan, xanthan gum, locust bean gum and gum arabic, waxes,polyacrylamide , mixtures thereof, and whatsoever.

Lubricants may be, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof or whatsoever.

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose, lower alkylsubstituted hydroxypropyl cellulose, polacrilin potassium, starch, pregelatinized starch, sodium alginate, mixtures thereof or whatsoever.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehyde , mixtures thereof or whatsoever.

Coating agents are, but not limited to, ethyl cellulose ,methyl cellulose, hydroxypropyl-methyl cellulose, hydroxyethyl cellulose, hydroxypropyl cellulose,polyvinylalcohol, polyvinyl acetate , cellulose acetate ,cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers ,polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose,polyvinyl pyrollidone,polyethylene glycol,cellulose trimellitate,hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof and the like .

Pore formers are, but not limited to, polyethylene glycol, camphor, polyvinylpyrrolidone ,dextrose,mannose, fructose,sucrose, glucodifructose, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, mixtures thereof and the like.

Organic solvents, used in preparation of solution are ,but not limited to, alcohols such as methanol, ethanol, propyl alcohol, isopropyl alcohol, 2-methyl-1-propanol etc. ;ketones such as acetone, methyl ethyl ketone, methyl isobutyl ketone etc. ; esters such as methyl acetate, ethyl acetate, propyl acetate, isopropyl acetate, ethyl formate etc. ; hydrocarbons such as heptane ; halogenated hydrocarbons such as dichloromethane.

Sweeteners are but not limited to, corn syrup,dextrose, invert sugar, fructose, saccharin, aspartame, acesulfame-K, Stevia rebaudiana, sucralose, sorbitol, mannitol, zylitol, mixtures thereof and the like

Other ingredients such as colorants and titanium dioxide can also be used.

In case of tablet is preferred, it may comprise sugar-based excipients, super-disintegrating agents, effervescent agents and further suitable excipients.

The formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods. Direct compression and wet granulation in fluid bed granulator are preferred.

Mozavaptan or a pharmaceutically acceptable salt thereof can be in microparticulate form with one or more excipients. Microparticulation can also be used as taste-masking method.

In another aspect, newly developed formulations results proved improved content uniformity.

The following examples are given for the purpose of illustration of the present invention and should not limit the scope of the invention.

### Example1: Orally Disintegrating Tablet Unit Formula of Mozavaptan

**Table 1**

| **Ingredients** | **mg/tablet** | **% (w/w)** |
|---|---|---|
| Mozavaptan | 30.00 | 25.00 |
| Mannitol | 28.1 | 23.42 |
| Polyplasdone XL 10 | 24.00 | 20.00 |
| Microcrystalline cellulose (Avicel pH 102) | 12.00 | 10.00 |
| Strawberry Flavour | 12.00 | 10.00 |
| Aspartame | 6.00 | 5.00 |
| Talc | 0.60 | 0.50 |
| Taste Masking agent | 4.80 | 4.00 |
| Glycerol Behenate | 2.50 | 2.08 |
| **Total Tablet Weight** | **120.00** | **100.00** |

### Example 2: Manufacturing Process of Example 1

Pharmaceutical formulation may be prepared using methods as below without limiting scope of this invention;
1. Diluent (mannitol), binder and glidant(talc) are loaded and mixed.
2. Sieved mixture of disintegrant(Polyplasdone XL 10), mozavaptan hydrochloride, sweetener(Aspartame) and flavour(Strawberry Flavour) are added to mixture obtained from step (1),
3. Obtained powder mixture in step (2) is blended
4. Sieved lubricant is added to the blended powder mixture obtained in step (3),
5. Final powder mixture, obtained in step (4), is blended.
6. The prepared final mixture is compressed with hardness range 20-75 N.
The average tablet hardness is preferably 35 N.

### Example 3: Effervescent Tablet Unit Formula of Mozavaptan

**Table 2**

| **Ingredients** | **mg/tablet** | **% (w/w)** |
|---|---|---|
| Mozavaptan | 30.00 | 6.67 |
| Sodium Bicarbonate | 118.80 | 26.4 |
| Citric Acid Anhydrous | 150.00 | 33.33 |
| Sucralose | 25.00 | 5.56 |
| Aspartame | 20.00 | 4.44 |
| Maltodextrin | 61.2 | 13.6 |
| Orange Flavour | 30.00 | 6.67 |
| Sodium Stearyl Fumarate | 15.00 | 3.33 |
| **Total Tablet Weight** | **450.00** | **100.00** |

### Example 4: Manufacturing Process of Example 3

Pharmaceutical formulation may be prepared using methods as below without limiting scope of this invention;

1. Mozavaptan HCl and maltodextrin is mixed and granulated with a suitable solvent.

2. The granules obtained from (1) are dried and granules obtained from step (1) are sieved with a suitable sieve.

3.Sweeteners (sucralose, sodium saccharin), and flavour (orange flavour) are mixed and added to powder mixture prepared in step(2)

4. Basic effervescent agent (potassium bicarbonate) and acidic effervescent agent (citric acid anhydrous) are added to powder mixture in step (3) and mixed.

5. Optionally, powder mixture in step (4) is sieved through suitable sized sieve.

6. Lubricant (sodium stearyl fumarate) is added and mixed.

7. Effervescent tablets are compressed with suitable punches having average target weight 450 mg/tablet.

### Example 5: Granules in Sachet Unit Formula of Mozavaptan

**Table 3**

| **Ingredients** | **mg/sachet** | **% (w/w)** |
|---|---|---|
| Mozavaptan | 30.00 | 10.00 |
| Sucralose | 35.00 | 11.66 |
| Saccharin | 10.00 | 3.34 |
| Mannitol | 175.00 | 58.33 |
| Cherry Flavour | 50.00 | 16.67 |
| **Total Tablet Weight** | **300.00** | **100.00** |

### Example 6: Manufacturing Process of Example 5

Pharmaceutical formulation may be prepared using methods as below without limiting scope of this invention;

1. Mozavaptan is diluted geometrically with diluent (mannitol) to obtain homogeneous mixture.

2. Sweeteners (sucralose and saccharin) are dissolved in alcohol.

3. Powder mixture in step (1) is granulated with solution in step (2) using fluid bed granulator.

4. Drying is continued until loss on drying is less than 1.5% (w/w).

5. Flavour (cherry flavour) is diluted geometrically with dry granules to obtain homogeneous mixture.

6. Diluted flavour (cherry flavour) is added to remaining dry granules and mixed.

7. Mixture in step (6) is filled into suitable packaging material, such as sachets, having average target powder weight of 300 mg per package.

## Claims

1. An orally disintegrating tablet comprising (i) an effective amount of mozavaptan and (ii) a pharmaceutically acceptable excipient or mixtures of excipients.

2. According to claim 1, the orally disintegrating tablet dosage form of mozavaptan is disintegrated in oral cavity (a) in less than three minutes; (b)more preferably in less than two minutes; (c)most preferably in less than one minute; (d)preferably in less than 30 seconds.

3. The orally disintegrating tablet, as claimed in preceding claims comprises;
mozavaptan or pharmaceutically acceptable salt thereof is in the range of from 1 % to 60, binder is in the range of from 0.1 % to 15 %, coating agent is in the range of from 0.1 % to 50 %, pore former is in the range of from 0.01 % to 5 %, disintegrant is in the range of from 10 % to 90 %, sweetener is in the range of from 0.1 % to 40 %, stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, by weight.

4. An effervescent tablet dosage form comprising (i) an effective amount of mozavaptan and (ii) a pharmaceutically acceptable excipient or mixtures of excipients.

5. The effervescent tablet, as claimed in claim 4, is administered as a solution in which drug is suspended or dispersed or dissolved in said solution.

6. The effervescent tablet, as claimed in claims from 4 to 5, comprises;
mozavaptan or pharmaceutically acceptable salt thereof is in the range of from 1 % to 60 %, binder is in the range of from 0.1 % to 15 %, diluent is in the range of from 10 % to 90 sweetener is in the range of from 0.1 % to 40 %, stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, acidic and basic effervescent agents are in the range of from 0.1 % to 80%, disintegrant is in the range of from 0.5 % to 30 % by weight of pharmaceutical dosage form.

7. A unit dose of granules in sachet comprising (i) an effective amount of mozavaptan and (ii) a pharmaceutically acceptable excipient or mixtures of excipients.

8. The sachet formulation, as claimed in claim 7, includes powder, granulate, pellets, spheres, particles or effervescent powder, effervescent granulate, effervescent pellets, effervescent spheres, effervescent particles or mixtures thereof.

9. The sachet formulation, as claimed in claims from 7 to 8 comprises:
mozavaptan or pharmaceutically acceptable salt thereof is in the range of from 1 % to 60 %, binder is in the range of from 0.1 % to 15 %, diluents is in the range of from 10 % to 90 %, sweetener is in the range of from 0.1 % to 40 %,
stabilizing agent is in the range of from 0.1 % to 5 %, flavour is in the range of from 0.1 % to 15 %, glidant is in the range of from 0.1 % to 10 %, lubricant is in the range of from 0.1 % to 20 %, surfactant is in the range of from 0.1 % to 10 %, disintegrant is in the range of from 0.5 % to 30 % by weight of pharmaceutical dosage form.

10. A dry syrup formulation comprising mozavaptan or pharmaceutically acceptable salt thereof and an excipient or mixtures of excipients.

11. Dry syrup formulation, as claimed in claim 11, comprises dry powder or granules or pellets or mixtures thereof.
